# EUROPEAN PATENT APPLICATION

(11) **EP 3 747 503 A1**
(43) Date of publication of application: **09.12.2020**
(21) Application number: 19774933.6
(22) Date of filing: 18.02.2019
(51) Int. Cl.: A61N 1/36

(54) **NECK MASSAGER**

(30) Priority: 29.03.2018 CN 201810276125; 29.03.2018 CN 201820443366 U
(71) Applicant: WEAR FUTURE TECHNOLOGIES CO., LTD, Shenzhen, Guangdong 518000 (CN)
(72) Inventor: LIU, Jie, Shenzhen, Guangdong 518000 (CN); WANG, Zhiguo, Shenzhen, Guangdong 518000 (CN); DENG, Ligang, Shenzhen, Guangdong 518000 (CN); XIAO, Hua, Shenzhen, Guangdong 518000 (CN)
(74) Representative: karo IP
(86) International application number: PCT/CN2019/075308
(87) International publication number: WO 2019/184615

(57) **Abstract**

A neck massaging device includes an elastic support, a silicone rubber base arranged on the elastic support. An electrode plate disposed on a side of the silicone rubber base facing away from the elastic support massages a neck of a wearer.

## Description

The present disclosure claims priority to the Chinese Patent Application No. 201810276125.5, filed to the **Patent Office of the People's Republic of China** on 29 March, 2018, and entitled **"NECK MASSAGING DEVICE",** which is incorporated in the present disclosure by reference in its entity.

The present disclosure claims priority to the Chinese Patent Application No. 201820443366.X, filed to the **Patent Office of the People's Republic of China** on 29 March, 2018, and entitled **"NECK MASSAGING DEVICE",** which is incorporated in the present disclosure by reference in its entity.

### Field

The present disclosure relates to the field of medical care and, more particularly, to a neck massaging device.

### BACKGROUND

In recent years, due to work and bad living habits, cervical spondylosis has youth-oriented tendency, and more and more people suffers from cervical spondylosis. As an effective non-surgical neck healthcare measure, neck massaging devices are selected by many patients suffering from cervical spondylosis.

A neck massaging device in the art generally performs massage in an electric pulse way. An electric pulse acting on a human body is conducted by electrode plates which are fixed on the neck massaging device. However, the positions of the electrode plates cannot be adjusted according to the using condition of the user. Therefore, comfortableness level of the user is affected accordingly.

### SUMMARY

The present disclosure is mainly aimed to provide a neck massaging device to solve the technical problem that positions of electrode plates of a traditional neck massaging device cannot be adjusted as required.

According to an embodiment of the present disclosure, a neck massaging device may be provided, which may include an elastic support, a silicone rubber base, the silicone rubber base may be arranged on the elastic support, and an electrode plate. The electrode plate may be disposed to the silicone rubber base and arranged on a side faced away from the elastic support for massaging the neck of a wearer.

Alternatively, the silicone rubber base may be of a hollow structure; and a projecting area on the elastic support of a part of the silicone rubber base that joins the elastic support may be larger than a projecting area on the elastic support of another part of the silicone rubber base that joins the electrode plate.

Alternatively, the thickness of a wall of the silicone rubber base may reduce gradually in a direction from the electrode plate to the elastic support.

Alternatively, the neck massaging device may further include a mounting seat and a massage head, the mounting seat includes the silicone rubber base and a fixing table arranged on the silicone rubber base, and a massage head disposed on the fixing table and arranged at a side of the fixing table faced away from the silicone rubber base. The electrode plate may be disposed on a side of the massage head faced away from the fixing table.

Alternatively, an accommodating space may be defined in the fixing table, and a connecting shaft may be disposed on a side of the massage head, which is faced away from the electrode plate. The connecting shaft may be arranged in the accommodating space, with one end connected to the silicone rubber base, and the other end fixed on the massage head.

Alternatively, the fixing table may be provided with a protrusion portion protruding towards the silicone rubber base, in which the accommodating space is formed. A locking member may be disposed in an inner side of the silicone rubber base, which is connected and locked to the connecting shaft.

Alternatively, a fixing tray may be disposed on the fixing table, and a recessed groove fitting with the fixing tray may be formed in the massage head.

Alternatively, a cross-sectional area of the fixing tray may be larger than that of the protrusion member, and the recessed groove may be arranged around the connecting shaft.

Alternatively, a positioning piece may be disposed on a side of the fixing tray facing the massage head, and a positioning groove which is adapted to fit with the positioning piece may be formed in the recessed groove.

Alternatively, the distance between the two electrode plates may range from 5 mm to 25 mm.

Alternatively, the neck massaging device may further include two clamping arms and silicone rubber layers. The two clamping arms may be fixing to each end of the elastic support respectively, which are arranged oppositely and located at the same side of the elastic support, and two silicone rubber layers may be arranged respectively on sides of the two clamping arms facing with each other.

Alternatively, a damping sponge may be disposed in each clamping arm, and arranged at a side of the clamping arm close to the silicone rubber layer.

Alternatively, each clamping arm may further include a first shell and a second shell. The first shell may be arranged at a side close to each silicone rubber layer, and the second shell may be arranged at the side faced away from the silicone rubber layers. The edges of the first shell and the second shell are connected with each other to form a hollow cavity.

Alternatively, each clamping arm may further include a supporting seat for the damping sponge arranged in the cavity, and the damping sponge may be disposed on one side of the supporting seat for the damping sponge.

Alternatively, the supporting seat for the damping sponge may be disposed on the second shell.

According to the neck massaging device of the present disclosure, by arranging the electrode plate on the massage head, and then mounting the massage head to the mounting seat, due to the softness of the silicone rubber base itself, the angle and position of the massage head may be adjusted according to an appropriate position of user's neck. Therefore, the electrode plate may be always fit to user's neck, so that the technical problem that the position of the electrode plate of the traditional neck massaging device may not be adjusted as required is solved.

### BRIEF DESCRIPTION OF THE DRAWINGS

In order to explain the technical solutions in the embodiments of the present disclosure or the prior art more clearly, the drawings used by the description of the embodiments or the prior art will be briefly introduced below. Obviously, the drawings in the following description may be merely some embodiments of the present disclosure. For those of ordinary skilled in the art, other drawings may be obtained according to the structures shown in the drawings without creative effort, in which:
Fig. 1 is a schematic view of a neck massaging device according to an embodiment of the present disclosure;
Fig. 2 is an exploded view of the neck massaging device shown in Fig. 1;
Fig. 3 is a schematic partial sectional view of the neck massaging device shown in Fig. 1;
Fig. 4 is a schematic view of a neck massaging device according to another embodiment of the present disclosure;
Fig. 5 is an exploded view of a first clamping arm as shown in Fig. 4;
Fig. 6 is an exploded view of a second clamping arm as shown in Fig. 4;

The purpose realization, functional characteristics and advantages of the present disclosure will be further described with reference to the drawings in combination with the embodiments as described hereinafter.

### DETAILED DESCRIPTION

In the following, the technical solutions in the embodiments of the present disclosure will be clearly and completely described with reference to the drawings in the embodiments of the present disclosure. Obviously, the described embodiments may be only a part of the embodiments of the present disclosure, but not all of the embodiments. Based on the embodiments of the present disclosure, all other embodiments obtained by those of ordinary skilled in the art without creative effort shall fall within the protection scope of the present disclosure.

It should be noted that all directional indications (such as up, down, left, right, front, and rear ......) in the embodiments of the present invention are only used to explain the relative position relationship between the components, the movement situation, and the like in a specific posture (as shown in the drawings), and if the specific posture is changed, the directional indication is changed accordingly.

In addition, descriptions such as "first" and "second" in the present disclosure may be for descriptive purposes only, and cannot be understood as indicating or implying the relative importance or implicitly indicating the number of indicated technical features. Therefore, the features defined as "first" and "second" may explicitly or implicitly include at least one of the features. In the description of the present disclosure, the meaning of "a plurality of" may be at least two, for example, two, and three and so on, unless it may be specifically defined otherwise.

According to the present disclosure, the terms "connection", "fixing" and the like should be understood in a broad sense unless otherwise specified and defined, for example, "fixing" may be fixed connection or detachable connection or an integral whole, may be mechanical connection or electrical connection, may be direct connection or indirect connection through an intermediate medium, and may be internal connection of two elements or interaction between two elements, unless it may be clearly defined otherwise. For those of ordinary skilled in the art, the specific meanings of the above terms in the present disclosure may be understood according to specific situations.

In addition, the technical solutions between the various embodiments of the present disclosure may be combined with each other, but must be based on what may be achieved by those of ordinary skilled in the art. When the combination of technical solutions conflicts or cannot be achieved, such a combination of technical solutions should be considered to be nonexistent and not within the scope of protection claimed by the present disclosure.

A neck massaging device according to an embodiment of the present disclosure will be described.

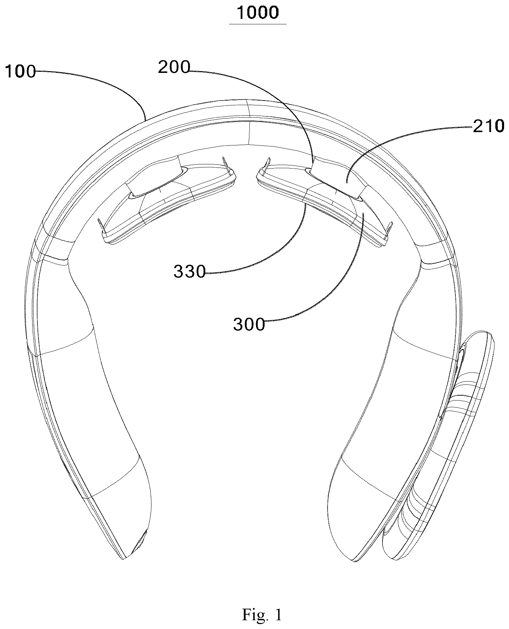

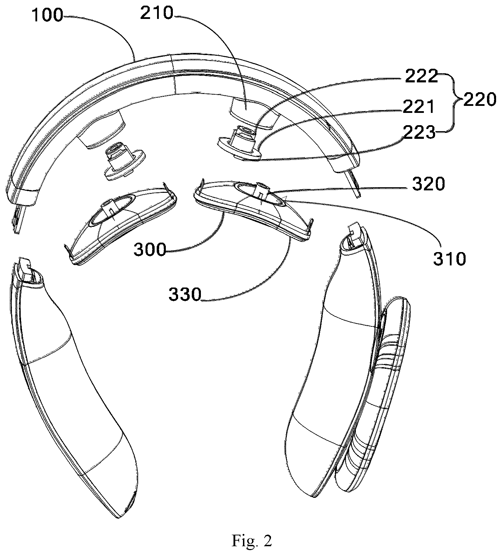

As shown in Figs. 1-3, the neck massaging device may include an elastic support 100, a silicone rubber base 210 and an electrode plate 330. The silicone rubber base 210 may be disposed on the elastic support 100. The electrode plate 330 may be disposed on a side of the silicone rubber base 210 faced away from the elastic support 100 for massaging the neck of a wearer.

According to the neck massaging device of the present disclosure, by arranging the electrode plate 330 on a side of the silicone rubber base 210 faced away from the elastic support 100, due to the softness of the silicone rubber base itself, the electrode plate may contact the neck of the wearer smoothly, so that the problem for adjusting the position of the electrode plate 330 in the conventional neck massaging device may be solved accordingly.

According to an embodiment of the present disclosure, the electrode plate 330 may be disposed on the massage head 300, and then the massage head may be mounted to the mounting seat 200. Therefore, the angle and position of the massage head 300 may be adjusted according to an appropriate position of user's neck.

Specifically, the silicone rubber base 210 may be of a hollow structure. A projecting area on the elastic support 100 of a part of the silicone rubber base 210 that joins the elastic support 100 may be larger than a projecting area on the elastic support 100 of a side of the silicone rubber base 210 that joins the electrode plate 330. The silicone rubber base 210 may be a hollow structure with a ladder-like shape, by which the silicone rubber base 210 may conveniently rotate at certain angle.

Specifically, the thickness of a wall 211 of silicone rubber base 210 may reduce gradually in a direction from the electrode plate to the elastic support 100. The silicone rubber wall 211, close to the elastic support 100, of the silicone rubber base 210 may be relatively thinner; therefore, the resistance generated when the rotation of the silicone rubber base 210 is adjusted may be reduced.

Specifically, the neck massaging device may further include a mounting seat 200 and a massage head 300. The mounting seat 200 may include the silicone rubber base 210 and a fixing table 220. The fixing table 220 may be arranged on the silicone rubber base 210. The massage head 300 may be disposed on the fixing table 220 and arranged at a side of the fixing table 220 faced away from the silicone rubber base 210. The electrode plate 330 may be disposed on a side of the massage head 300 faced away from the fixing table 220. The silicone rubber base 210 may be made of soft materials, and it is difficult to detachably arrange the massage head 300 on the mounting seat 200. Accordingly, the hard fixing table 220 may be disposed. The fixing table 220 may be bonded with the silicone rubber base 210 by glue, and then, the massage head 300 may be detachably connected on the fixing table 220.

Specifically, an accommodating space 224 may be defined in the fixing table 220. A connecting shaft 320 may be disposed on a side of the massage head 300, which is faced away from the electrode plate 330. The connecting shaft 320 may be arranged in the accommodating space 224. One end of the connecting shaft 320 may be connected to the silicone rubber base 210, and the other end thereof fixed on the massage head 300. The massage head 300 may be mounted to the mounting seat 200 through the connecting shaft 320. Due to the softness of the silicone rubber base 210 itself, the rotation of the massage head 300 may be adjusted without dead angles within the range of 360 degree.

Specifically, the fixing table 220 may be provided with protrusion portion 222 protruding towards the silicone rubber base 210, in which the accommodating space 224 may be formed. The inner sides of the silicone rubber base 210 are provided with a locking member 230, which is connected and locked to the connecting shaft 320. The locking member 230 may be a locking nut. And the connecting shaft 320 may be provided with a screw thread matched with the locking nut.

Specifically, a fixing tray 221 may be disposed on the fixing table 220. A recessed groove 310 fitting with the fixing tray 221 may be formed in the massage head 300. The fixing tray 221 may be engaged to the recessed groove 310, so that the massage head 300 may be located and mounted on the mounting seat 200.

Specifically, the cross-sectional area of the fixing tray 221 may be larger than the area of protrusion member 222, and the recessed groove 310 may be arranged around the connecting shaft 320. Alternatively, the protrusion member 222 may be vertically disposed on the fixing tray 221. The fixing tray 221 may be fixed to the end of the silicone rubber base 210, which is faced away from the elastic support 100.

Specifically, a positioning piece 223 may be disposed on a side of the fixing tray 221 facing the massage head 300. A positioning groove (not shown) which is adapted to fit with the positioning piece 223 may be formed in the recessed groove 310. The positioning groove is formed in the recessed groove 310. The positioning pieces 223 may be engaged with the positioning groove, so that the massage head 300 may be precisely mounted on the mounting seat 200.

Specifically, the distance between the two electrode plates 330 ranges from 5 mm to 25 mm.

### Embodiment 2:

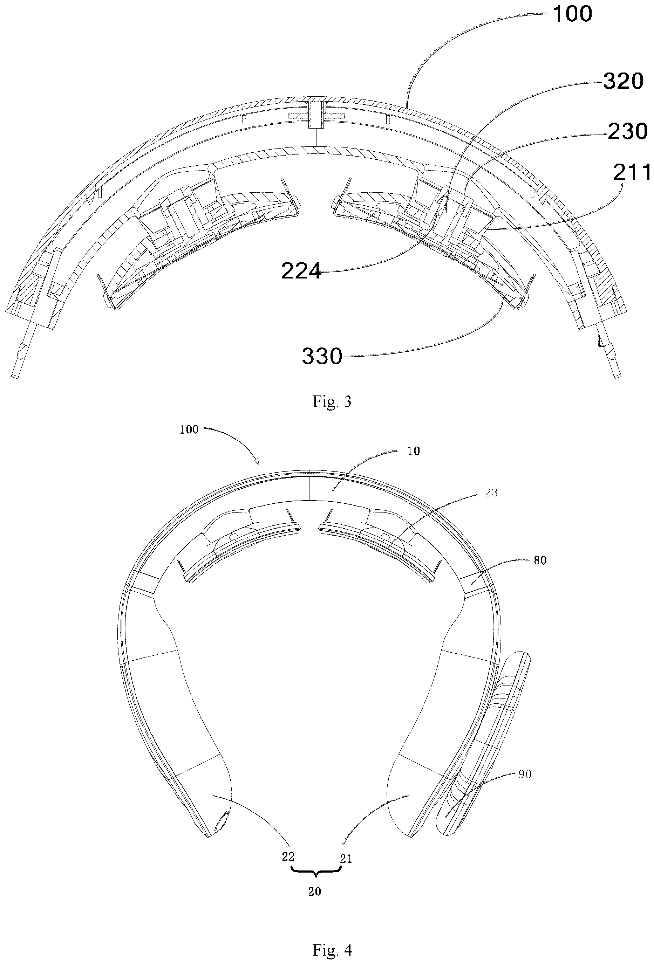

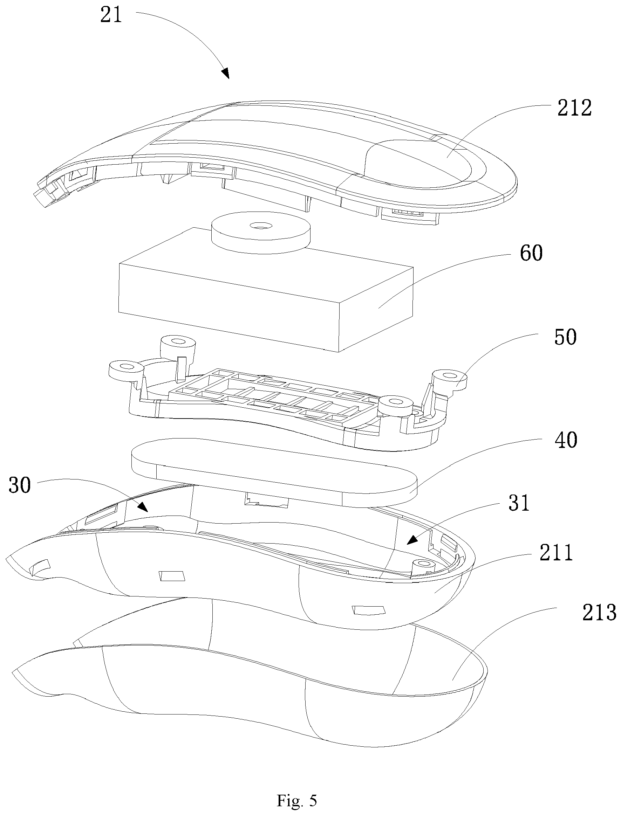

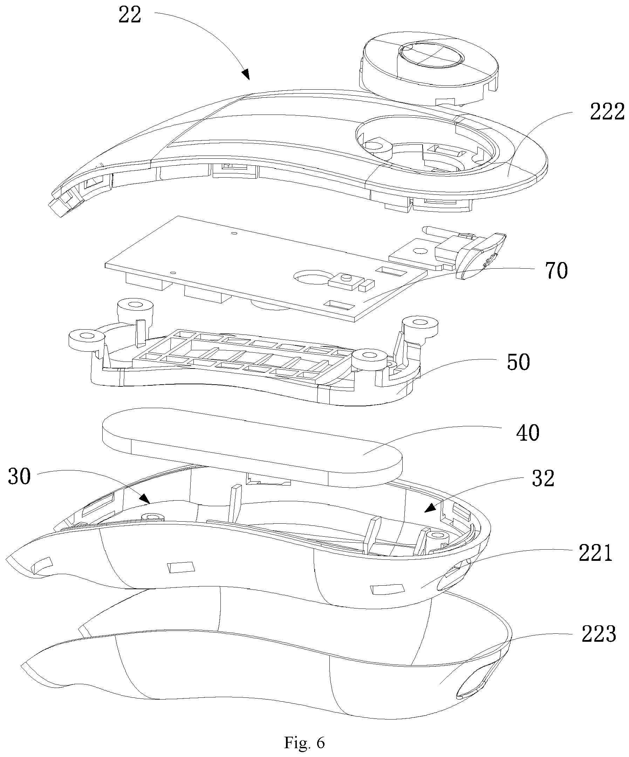

As shown in Fig. 4 to Fig. 6, the neck massaging device 100 according to a second preferred embodiment of the present disclosure may include an elastic support 10, two clamping arms 20 and electrode plates 23 arranged on the elastic support 10. The two ends of the elastic support 10 may be fixedly connected with the two clamping arms 20 respectively. The two clamping arms 20 may be arranged oppositely and located at the same side of the elastic support 10. The neck massaging device 100 may further include silicone rubber layers. The silicone rubber layers may be arranged respectively on sides of the two clamping arms 20 facing with each other.

According to the neck massaging device 100 of the present disclosure, the neck massaging device 100 may be worn by virtue of the deformation of the elastic support 10, so as to well adapt to the dimension of a neck on the premise that an electric pulse generating device is not damaged. In addition, the deformation of the elastic support 10 may depend on the form change of the elastic of the elastic support 10 itself, rather than the high non-deformable elastic force of the springs. Therefore, the elastic force of the clamping arms 20 integrally clamped on the neck is softer and more comfortable. Moreover, the neck massaging device 100 further includes silicone rubber layers which are arranged at the opposite sides of the two clamping arms 20 and may fit to the neck of a user according to the shape of the neck of the user. Therefore, compared with a plastic material in the prior art, a silicone rubber material may increase the friction force between the clamping arms 20 and the neck skin of a wearer when being in contact with the skin, so that it is beneficial for the neck massaging device 100 to swing with swing of the neck of the wearer, namely the neck massaging device 100 may be more comfortably and better attached to the neck of the wearer, and the acmesthesia feeling caused by the electrode plates 23 occasionally when detaching from the skin may be avoided.

According to the neck massaging device 100 of the present disclosure, by including the elastic support 10 and the two clamping arms 20, connecting the two ends of the elastic support 10 to the two clamping arms 20 respectively, and arranging the two clamping arms 20 at the same side of the elastic support 10, a recessed structure may be formed by the elastic support 10 and the two clamping arms 20. In that case, the neck massaging device 100 may be worn and positioned by attachment of the clamping arms 20 and the neck of the user. In addition, by arranging silicone rubber layers at the opposite sides of the two clamping arms 20, the silicone rubber layers may be attached and fit to the neck of the user according to the shape of the neck of the user, so that the falling of the neck massaging device 100 may be avoided, which may be caused by that the clamping arms 20 may not be tightly attached to the neck of the user under the condition that the neck massaging device 100 vibrates or the user moves.

In the present embodiment, the neck massaging device 100 may include the elastic support 10, the two clamping arms 20 and the electrode plates 23 arranged on the elastic support. The two ends of the elastic support 10 may be fixedly connected with the two clamping arms 20 respectively. The two clamping arms 20 may be arranged oppositely and are located at the same side of the elastic support 10. In this case, a recessed structure may be formed by the elastic support 10 and the two clamping arms 20, and the size of an opening of the recessed structure may be changed by virtue of elastic deformation of the elastic support 10. So that the neck massaging device 100 may be adapted to the neck dimensions of different users. Further, by arranging silicone rubber layers which are arranged at the opposite sides of the two clamping arms 20, when the user wears the neck massaging device 100, the soft silicone rubber layer may be tightly attached to the skin. Accordingly the problem that the clamping arms 20 made of metals or plastics are not firmly attached to the neck may be avoided.

According to an embodiment of the present disclosure, a damping sponge 40 may be disposed in each clamping arm 20, and arranged at a side of the clamping arm 20 close to the silicone rubber layer. The damping sponge 40 may be configured to limit the relative movement of elements inside the clamping arms 20 and reduce the vibration of the elements in the clamping arms 20. In an alternative embodiment, when the neck massaging device 100 is operated, it is possible that the position of the neck massaging device 100 may be changed by the vibration of the neck massaging device 100 or a movement of the user. In that case, the damping sponge 40 may reduce influences of the internal elements to the clamping arms 20 and reduce the vibration of the clamping arms 20.

Specifically, each clamping arm 20 may further include a first shell (not shown) and a second shell (not shown). The first shell may be arranged at a side close to each silicone rubber layers. The second shell may be arranged at the side faced away from each silicone rubber layer. The edges of the first shell and the second shell are connected with each other, therefore a hollow cavity 30 may be formed.

Specifically, the clamping arms 20 may further include a supporting seat 50 for the damping sponge 40 arranged in the cavity. The damping sponge 40 may be disposed at one side of the supporting seat 50. In an alternative embodiment, the supporting seat 50 for the damping sponge may be configured to fix the damping sponge 40. At least one edge protrusion may be disposed on one side of the supporting seat 50 for the damping sponge 40, which may be facing the damping sponge 40, which may be configured to limit the position of the damping sponge 40 and prevent the damping sponge 40 from displacing.

Specifically, the supporting seat 50 for damping sponge 40 may be arranged on the second shell. In an alternative implementation, the supporting seat 50 for damping sponge 40 may be connected to the second shells in a buckling or bolt way.

Specifically, the neck massaging device 100 may further include a battery 60 and a control motherboard 70. The clamping arms 20 may include a first clamping arm 21 and a second clamping arm 22. The first clamping arm 21 may include a first shell 211, a second shell 212 and a first silicone rubber layer 213. The second clamping arm 22 includes a third shell 221, a fourth shell 222 and a second silicone rubber layer 223. The cavities 30 may include a first cavity 31 and a second cavity 32. In an alternative implementation, the battery 60 and the control motherboard 70 may be respectively accommodated in the two clamping arms 20. That is, the battery 60 may be accommodated in the first cavity 31, and the control motherboard 70 may be accommodated in the second cavity 32. Therefore the weights of the two clamping arms 20 may be well balanced, accordingly when the user uses the neck massaging device 100, the neck pressing non-uniformity of the clamping arms 20 may be avoided, and the user experience is improved.

Specifically, the battery 60 and the control motherboard 70 may be arranged at the other side respectively corresponding the supporting seat 50 for the damping sponge 40. In an alternative implementation, in each clamping arm, the supporting seat 50 for the damping sponge 40 may be connected with the second shell. The battery 60 may be arranged in the cavity (not indicated) defined by the second shell 212 and the respective supporting seat 50. The control motherboard 70 may be arranged in the cavity (not indicated) defined by the second shell 222 and the respective supporting seat 50. The connection of the second shells and the supporting seats 50 for the damping sponge 40 plays a role in limiting the position of the battery 60 or the control motherboard 70.

Specifically, the shape of the second shells and the silicone rubber layers are the same. According to one embodiment of the present disclosure, the shape of the second shells may be same as that of the silicone rubber layers, so that the silicone rubber layer may be supported by the second shell. Therefore, the serious deformation of the silicone rubber layers, and thus clamping instability, may be avoided when clamping the users' neck.

Alternatively, the silicone rubber layer may be coated with hand feeling oil, so that the hand feeling of silicone rubber sleeves of the clamping arms may be enhanced, the wear resistance of the silicone rubber layer improved, and meanwhile, the appearance effect of the neck massaging device 100 improved.

According to an embodiment of the present disclosure, the neck massaging device 100 may further include a connecting piece 80, which may be configured to connect the elastic support 10 and the clamping arms 20. In an alternative implementation, the connecting pieces 80 may be of fixed structures or telescopic structures. When the connecting pieces 80 are of the fixed structures, the clamping dimension of the neck massaging device 100 may be changed by the elastic deformation of the elastic support 10. When the connecting pieces 80 are of the telescopic structures, the distances between the elastic support 10 and the clamping arms 20 of the neck massaging device 100 may be changed through the connecting pieces 80. Meanwhile, the clamping dimension of the neck massaging device 100 may be changed by the telescopic length of the connecting pieces 80 and the elastic deformation of the elastic support 10.

Alternatively, the first shell may be integrated or detachable with the silicone rubber layer. If the first shell is detachable with the silicone rubber layer, the silicone rubber layer may be connected with the first shell in a buckling way. If the first shell is integrated with the silicone rubber layer, the silicone rubber layer may be connected with the first shell in a bonding way.

According to an embodiment of the present disclosure, the neck massaging device 100 may further include a remote control terminal. In an alternative implementation, the remote control terminal may be a mobile terminal (not shown in the figure) or a remote controller 90. If the remote control terminal is the mobile terminal, the mobile terminal may be internally provided with an application program correlated to the neck massaging device 100. In this case, the working state of the neck massaging device 100 may be controlled by the mobile terminal through the application program. When the remote control terminal is the remote controller 90, a magnet may be disposed in the first cavity 30 and close to the second shell 212, or in the second cavity 32 and close to the fourth shell 222. Correspondingly, a magnet attraction plate (not shown) may be disposed in the remote controller 90. The remote controller 90 may be attracted to the second shell 212 or the fourth shell 222 under the action of a magnetic force. In addition, a control circuit board (not shown) and a control button (not shown) may be arranged on the remote controller 90, so that the starting and stopping of the neck massaging device 100 may be controlled through the control button.

The above descriptions may be only preferred embodiments of the present disclosure, and thus do not limit the patent claims of the present disclosure. Any equivalent structural transformation made by using the description and drawings of the present disclosure under the concept of the present disclosure, or direct/indirect application in other related technical fields may be included in the patent protection scope of the present disclosure.

## Claims

1. A neck massaging device, comprising:
an elastic support and a silicone rubber base, the silicone rubber base is disposed on the elastic support; and an electrode plate disposed on a side of the silicone rubber base faced away from the elastic support for massaging the neck of a wearer.

2. The neck massaging device according to claim 1, wherein
the silicone rubber base is of a hollow structure; and a projecting area on the elastic support of a part of the silicone rubber base that joins the elastic support is larger than a projecting area on the elastic support of another part of the silicone rubber base that joins the electrode plate.

3. The neck massaging device according to claim 2, wherein the thickness of a wall of the silicone rubber base reduces gradually in a direction from the electrode plate to the elastic support.

4. The neck massaging device according to claim 2, further comprising
a mounting seat and a massage head, the mounting seat comprising the silicone rubber base and a fixing table arranged on the silicone rubber base; and the massage head disposed on the fixing table and arranged at a side of the fixing table faced away from the silicone rubber base; the electrode plate is disposed on a side of the massage head faced away from the fixing table.

5. The neck massaging device according to claim 4, wherein an accommodating space is defined in the fixing table; a connecting shaft is disposed on a side of the massage head, which is faced away from the electrode plate; and the connecting shaft is arranged in the accommodating space with one end connected to the silicone rubber base and the other end fixed on the massage head.

6. The neck massaging device according to claim 5, wherein the fixing table is provided with a protrusion portion protruding towards the silicone rubber base, in which the accommodating space is formed; wherein a locking member is disposed in an inner side of the silicone rubber base, which is connected and locked to the connecting shaft.

7. The neck massaging device according to claim 6, wherein a fixing tray is disposed on the fixing table, and a recessed groove fitting with the fixing tray is formed in the massage head.

8. The neck massaging device according to claim 7, wherein a cross-sectional area of the fixing tray is larger than that of the protrusion member; wherein
the recessed groove is arranged around the connecting shaft.

9. The neck massaging device according to claim 8, wherein a positioning piece is disposed on a side of the fixing tray facing the massage head, and a positioning groove for fitting with the positioning piece is formed in the recessed groove.

10. The neck massaging device according to claim 1, wherein the distance between the two electrode plates ranges from 5 mm to 25 mm.

11. The neck massaging device according to claim 1, **characterized in that** the neck massaging device further comprising two clamping arms and two silicone rubber layers, the two clamping arms are fixing to each end of the elastic support respectively, which are arranged oppositely and located at the same side of the elastic support; and the two silicone rubber layers are arranged respectively on sides of the two clamping arms facing with each other.

12. The neck massaging device according to claim 1, wherein a damping sponge is disposed in each clamping arm and arranged at a side of the clamping arm close to the silicone rubber layer.

13. The neck massaging device according to claim 12, wherein each clamping arm further comprises a first shell and a second shell, the first shell is arranged at a side close to each silicone rubber layer, and the second shell is arranged at the side faced away from each silicone rubber layer; wherein
edges of the first shell and the second shell are connected with each other to form a hollow cavity.

14. The neck massaging device according to claim 13, wherein each clamping arm further comprises a supporting seat for the damping sponge arranged in the cavity; wherein
the damping sponge is disposed on one side of the supporting seat for the damping sponge.

15. The neck massaging device according to claim 14, wherein the supporting seat for the damping sponge is disposed on the second shell.
